# EUROPÄISCHE PATENTSCHRIFT

(11) **EP 0 524 548 B1**
(45) Veröffentlichungstag und Bekanntmachung des Hinweises auf die Patenterteilung: **19.07.1995**
(21) Anmeldenummer: 92112181.0
(22) Anmeldetag: 16.07.1992
(51) Int. Cl.: A01N 31/04, A61K 7/00, A01N 31/02

(54) **Antimikrobiell wirksame Gemische**
Antimicrobial mixtures
Mélanges antimicrobiens

(30) Priorität: 25.07.1991 DE 4124664
(43) Veröffentlichungstag der Anmeldung: 27.01.1993
(73) Patentinhaber: Henkel Kommanditgesellschaft auf Aktien, 40191 Düsseldorf (DE)
(72) Erfinder: Waldmann-Laue, Marianne, Dr., W-4018 Langenfeld (DE); Slominski, Irina, W-4300 Essen 1 (DE); Stoll, Gerhard, Dr., W-4052 Korschenbroich 1 (DE); Meyer, Bernhard, Dr., W-4020 Mettmann (DE); Schneider, Werner, Dr., W-4150 Krefeld (DE)

(56) Entgegenhaltungen:
- EP-A- 0 166 611
- EP-A- 0 259 249
- DE-A- 4 026 756
- US-A- 2 255 916
- US-A- 3 502 594
- US-A- 3 652 764
- US-A- 4 321 257
- US-A- 5 104 447
- DATABASE WPI Week 7639, Derwent Publications Ltd., London, GB; AN 76-72925X(39)

## Beschreibung

Die Erfindung betrifft antimikrobiell wirksame Gemische mit einem Gehalt an antimikrobiell wirksamen Diolen und aromatischen Alkoholen.

Die antimikrobiellen Eigenschaften bestimmter aromatischer Alkohole, z. B. des Benzylalkohols, sind schon seit längerer Zeit bekannt (vgl. M.A.L. Mackie et al., Pharm. Acta Helv. 61, Nr. 12 (1986). Auch langkettige, lineare 1,2- und 1,3-Diole weisen gewisse keimhemmende Eigenschaften auf (vgl. Journal of Food Science Vol 42 (1977), No. 3, 699-706, DE-OS-22 04 943 und JP 76/91327, Chem. Abstr. 85, 117980r (1976)).

Die antimikrobiellen Eigenschaften dieser Stoffe sind jedoch nur schwach ausgeprägt, so daß eine sichere Konservierung mikrobiell verderblicher Zubereitungen nur mit untragbar hohen Konzentrationen dieser Stoffe möglich ist.

Auf dem Gebiet der Desinfektions- und Konservierungsmittel besteht aus Gründen der Umweltschonung, der physiologischen Verträglichkeit und der Wirtschaftlichkeit ein großes Bedürfnis nach antimikrobiellen Wirkstoffen und Wirkstoffkombinationen, die schon bei geringer Anwendungskonzentration eine hinreichende antimikrobielle Wirkung zeigen. Dies gilt ganz besonders für die Konservierung von Körperreinigungs- und pflegemitteln. Hier sind synergistisch wirkende Kombinationen, bekannter und physiologisch gut verträglicher Wirkstoffe von besonders hohem Wert.

Es wurde gefunden, daß Gemische, die
(A) mindestens einen antimikrobiell wirksamen Alkohol der Formel I 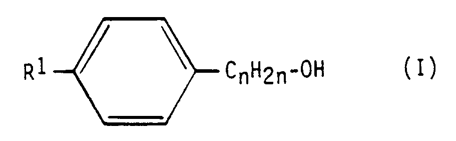 in der R¹ Wasserstoff oder eine Alkylgruppe mit 1 bis 4 C-Atomen und n eine ganze Zahl von 1 bis 6 ist und
(B) mindestens ein antimikrobiell wirksames 1,2- oder 1,3-Diol der Formel II

   R²-CHOH-(CHR³)ₓ-CH₂OH (II)

   enthalten,
   in der x = 0 oder 1 ist und wenn x = 0 ist, R² eine Alkylgruppe mit 6 bis 22 C-Atomen oder eine Alkoxymethyl- oder 2-Hydroxy-alkoxymethylgruppe mit jeweils 6 bis 22 C-Atomen in der Alkoxygruppe ist und wenn x = 1 ist, die Gruppe R² Wasserstoff ist und R³ eine der vorgenannten Bedeutungen von R² hat, wobei die Komponenten (A) und (B) im Gewichtsverhältnis (A):(B) = 9:1 bis 1:9 vorliegen, überraschend hohe antimikrobielle Wirkungen aufweisen, die sich nicht durch eine additive Wirkung der sehr wenig wirksamen Einzelkomponenten erklären lassen.

Solche Gemische sind Gegenstand der Erfindung und können z. B. als Konzentrate in einem geeigneten Träger formuliert werden und zur Desinfektion fester Oberflächen, zur Herstellung desinfizierender Reinigungsmittel oder zur Konservierung wäßriger Zubereitungen mikrobiell abbaubarer Stoffe eingesetzt werden.

Bevorzugt ist die Verwendung der erfindungsgemäßen Gemische zur Konservierung wäßriger Zubereitungen mikrobiell abbaubarer Stoffe.

Als Träger zur Konfektionierung der erfindungsgemäßen Gemische eignen sich z. B. niedere Alkohole wie Ethanol und Isopropanol oder Polyole wie 1,2-Propylenglycol, Glycerin oder flüssige Polyethylenglycole sowie deren Gemische oder deren Gemische mit Wasser.

Als antimikrobiell wirksame Alkohole der Formel I eigenen sich z. B. Benzylalkohol, Phenylethanol, Phenylpropanol, Phenylbutanol, Phenylpentanol und Phenylhexanol. Für kosmetische Produkte ergibt sich bei einigen der genannten Produkte der Vorteil, daß sie angenehm riechen und eine weitere Parfümierung entweder entbehrlich ist oder mit geringeren Mengen anderer Duftstoffe erzielt werden kann. Dies gilt vor allem für Alkohole der Formel I, in der n = 2 oder 3 ist, das heißt für Phenylethanol und Phenylpropanol (Hydrozimtalkohol), die daher für die Anwendung in Kosmetika bevorzugt sind.

Als antimikrobiell wirksame Diole der Formel II eigenen sich insbesondere 1,2-Alkandiole mit 8 bis 24 C-Atomen (x = 0, R² = C₆-C₂₂-Alkyl), 1,3-Alkandiole mit 9 bis 25 C-Atomen (x = 1, R² = C₆-C₂₂-Alkyl, R³ = H), Glycerinmonoalkylether (x = 1, R² = C₆-C₂₂-Alkoxymethyl, R³ = H), und Glycerinmono-(2-hydroxy)alkylether (x = 1, R² = 2-Hydroxy-C₆-C₂₂-alkoxymethyl, R³ = H). Weitere geeignete Diole sind die 2-substituierten 1,3-Propandiole (x = 1, R² = H, R³ = C₆-C₂₂-Alkyl, C₆-C₂₂-2-Hydroxyalkyl, C₆-C₂₂-Alkoxymethyl oder C₆-C₂₂-2-Hydroxyalkoxymethyl). Bevorzugt geeignet sind dabei solche Diole, die sich als hautfreundliche, kosmetische Ölkomponenten eignen und daher bei der Anwendung in Kosmetika eine zusätzliche, äußerst erwünschte Funktion aufweisen. Dies sind bevorzugt solche 1,2-Diole der Formel II, in der x = 0 und R² eine Alkyl- oder Alkoxymethylgruppe mit 8 bis 14 C-Atomen ist. Solche 1,2-Diole und ihre Herstellung sind z. B. von H. Rutzen in Fette, Seifen, Anstrichmittel 82 (1980), Nr. 1, S. 23 f, beschrieben und von R.R. Egan in Cosmetics and Perfumery 88, März 1973, 45-50 als sehr hautfreundliche Ölkomponenten bezeichnet worden.

Ein als Konservierungsmittel geeignetes Konzentrat läßt sich z. B. herstellen aus
10 bis 30 Gew.-% eines aromatischen Alkohols der Formel I
10 bis 30 Gew.-% eines Diols der Formel II
40 bis 80 Gew.-% eines oder mehrerer Polyole aus der Gruppe der 1,2-Propylenglycol, Glycerin und Polyethylenglycol mit einem mittleren Molekulargewicht von 200 bis 1000.

Besonders geeignet ist ein Konzentrat, bestehend aus
20 Gew.-% Phenylethylalkohol oder Hydrozimtalkohol
20 Gew.-% 1,2-Dodecandiol und
60 Gew.-% 1,2-Propylenglykol.

Die erfindungsgemäßen antimikrobiell wirksamen Gemische eignen sich sehr gut zur Herstellung antiseptisch wirksamer Hautreinigungsmittel. Bevorzugt geeignet sind sie jedoch zur Konservierung wäßriger Zubereitungen mikrobiell abbaubarer oder verderblicher Stoffe. Diese Zubereitungen können z. B. Haut- und Körperreinigungsmittel sein, bevorzugt jedoch handelt es sich dabei um kosmetische Emulsionen zur Reinigung und Pflege des Körpers mit einem Gehalt an mikrobiell abbaubaren Ölen, Fetten, Proteinen, Kohlenhydraten oder Derivaten davon. Diese Produkte enthalten zur Konservierung gegen durch Bakterien oder Pilze verursachten Verderb ein antimikrobiell wirksames, erfindungsgemäßes Gemisch in einer Menge, die einem Gehalt von 0,2 bis 5 Gew.-% der Summe der Komponenten (A + B) entspricht.

Ein Konzentrat, enthaltend z. B. 20 Gew.-% des aromatischen Alkohols der Formel I, 20 Gew.-% des Diols der Formel II und 60 Gew.-% eines niederen Polyols, z. B. 1,2-Propylenglycol wird in einer Menge von ca. 0,5 bis 12 Gew.-% der zu konservierenden Zubereitung zugesetzt, um eine ausreichende Konservierung zu erhalten.

Die folgenden Beispiele sollen den Erfindungsgegenstand näher erläutern.

### Beispiele

### Prüfung der Konservierungswirkung

### I Prüfemulsion

Es wurden Prüfemulsionen nach folgender Rezeptur hergestellt:

| | |
|---|---|
| Paraffinöl | 17,0 Gew.-% |
| Isopropylpalmitat | 2,0 Gew.-% |
| mikrokristallines Wachs | 2,7 Gew.-% |
| Arlacel^{(R)}186 (1) | 2,5 Gew.-% |
| Zinkum | 3,0 Gew.-% |
| Magnesiumsulfat | 0,5 Gew.-% |
| Glycerin | 3,0 Gew.-% |
| 1,2-Propylenglycol | 2,0 Gew.-% |
| Konservierungsmittel(kombination) | 2,0 Gew.-% |
| Wasser | 65,3 Gew.-% |

In die Emulsionen wurden die folgenden Produkte als Konservierungsmittel eingesetzt:
- A1:: Phenylethylalkohol
- A2:: Hydrozimtalkohol (3-Phenylpropanol-1)
- B1:: 1,2-Dodecandiol
- B2:: 1-(2-Hydroxydodecyloxy)-2,3-propandiol
Die Zusammensetzung des Konservierungsmittels in den Prüfemulsionen 1 bis 8 ist der Tabelle I zu entnehmen.

### II Belastungstest

Die Konservierung wurde in einem qualitativen Belastungstest mit Bakterien- und Pilzgemisch geprüft. Die Belastung erfolgte mit ≧ 10⁶ Bakterien/g Produkt und ≧ 10⁵ Pilzen/g Produkt. Die Belastungssuspension wurde 1 %ig zugegeben.

| | | |
|---|---|---|
| Testkeime: | | |
| Bakterien: | Staphylococcus aureus | ATCC 6538 |
| | Enterococcus faecium | ATCC 6057 |
| | Escherichia coli | ATCC 11229 |
| | Enterobacter aerogenes | DSM 30053 |
| | Pseudomonas aeruginosa | ATCC 15442 |
| Pilze: | Candida albicans | ATCC 10231 |
| | Aspergillus niger | ATCC 6275 |
| | Penicillium rubrum | CMI 113729 |
| | Trichoderma viride | BAM T21 |

Die belasteten Proben wurden homogenisiert und bei Raumtemperatur gelagert. Nach 1, 3, 7, 14 und 21 Tagen wurden Proben entnommen, ausgeimpft und inkubiert (Pilze auf Würzeagar und -bouillon bei 30 °C, Bakterien auf Standard I - nähragar und -bouillon bei 37 °C). In der Tabelle I ist die Lagerzeit angegeben, nach welcher keine lebens- bzw. vermehrungsfähigen Keime in einer Probe von 0,1 g mehr nachweisbar waren.

**Tabelle I**

| Beispiel | **1** | **2** | **3** | **4** | **5** | **6** | **7** | **8** | **9** | **10** |
|---|---|---|---|---|---|---|---|---|---|---|
| **Konservierung** | | | | | | | | | | |
| A1 Gew.-% | 2 | - | - | - | 1 | 1 | - | - | 1 | 1 |
| A2 Gew.-% | - | 2 | - | - | - | - | 1 | 1 | - | - |
| B1 Gew.-% | - | - | 2 | - | 1 | - | 1 | - | 1 | 1 |
| B2 Gew.-% | - | - | - | 2 | - | 1 | - | 1 | - | - |

| **Abtötungszeit (Tage)** | | | | | | | | | | |
|---|---|---|---|---|---|---|---|---|---|---|
| Pilze | > 21 | 22 | 21 | 21 | 14 | 7 | 7 | 14 | 7 | 7 |
| Bakterien | > 21 | > 21 | > 21 | > 21 | 3 | 3 | 3 | 1 | 1 | 21 |

Die Beispiele 1 bis 4 (Vergleichsbeispiele) zeigen, daß mit den Komponenten A und B alleine, auch bei gleicher Einsatzmenge nicht die hohe Wirkung der Kombination (A + B) der Beispiele 5 bis 8 erreicht wird.

Folgende Emulsionen wurden zusätzlich in die Konservierungsprüfung einbezogen

### Beispiel 8: O/W-Emulsion

| | |
|---|---|
| Lameform^{(R)}TGi (2) | 1,0 Gew.-% |
| Brij 30 | 1,0 Gew.-% |
| mikrokristallines Wachs | 0,5 Gew.-% |
| 1,3-Diisooctyl-cyclohexan | 10,0 Gew.-% |
| Cetiol SN (3) | 3,0 Gew.-% |
| Avocadoöl | 3,0 Gew.-% |
| Magnesium-Aluminium-Silikat (Veegum^{(R)}) | 3,6 Gew.-% |
| Xanthan Gummi | 1,8 Gew.-% |
| Citronensäure | 0,13 Gew.-% |
| 1,2-Dodecandiol | 1,0 Gew.-% |
| Phenylethylalkohol | 1,0 Gew.-% |
| Wasser | ad 100,0 Gew.-% |

Dieses Produkt wies im Belastungstest Abtötungszeiten von 1 Tag für Bakterien und 7 Tage für Pilze auf.

### Beispiel 10: Duschbad

| | |
|---|---|
| Fettalkohol-C_{12/14}-polyglycolether (2 EO) -sulfat, Na-Salz, 28 %ig in Wasser | 50,0 Gew.-% |
| Dehyton K (30 %ig in H₂O) (4) | 8,0 Gew.-% |
| Nutrilan H (5) | 2,0 Gew.-% |
| Cetiol HE (6) | 2,0 Gew.-% |
| Oleylalkohol-polyglycol(5EO)-ether | 2,0 Gew.-% |
| Mergnat^{(R)}550 (Quaternium 41) | 1,0 Gew.-% |
| 1,2-Dodencandiol | 1,0 Gew.-% |
| Phenylethylalkohol | 1,0 Gew.-% |
| Citronensäure | 0,11 Gew.-% |
| Wasser | ad 100,0 Gew.-% |

Dieses Produkt wies im Belastungstest Abtötungszeiten von 21 Tagen für Bakterien und 7 Tage für Pilze auf.

### Beispiel 11: Konservierungsmittelkonzentrat

| | |
|---|---|
| Phenylethylalkohol | 20 Gew.-% |
| 1,2-Dodencandiol | 20 Gew.-% |
| 1,2-Propylenglycol | 60 Gew.-% |

### Beispiel 11: Konservierungsmittelkonzentrat

| | |
|---|---|
| Hydrozimtalkohol | 20 Gew.-% |
| 1,2-Dodecandiol | 20 Gew.-% |
| 1,2 Propylenglycol | 60 Gew.-% |

Es wurden die folgenden Handelsprodukte verwendet:
(1) Arlacel 186: Gemisch aus Ölsäuremono/diglycerid (90 %) und 1,2-Propylenglycol (10 %)
(2) Lameform TGi: Polyglyceryl (3)-diisostearat
(3) Cetiol SN: Cetyl-/stearyl-isononanoat
(4) Dehyton K: R CO-N-(CH₂)₃-N(CH₃)₂-CH₂ COO⁽⁻⁾ (RCO = Kokosacyl), 30 %ig in Wasser
(5) Nutrilan H: Eiweißhydrolysat, Na-Salz (32 % Feststoff in Wasser)
(6) Cetiol HE: Glycerinpolyglycolether(7EO)-kokosfettsäureester

## Patentansprüche

1. Antimikrobiell wirksame Gemische, enthaltend
(A) mindestens einen antimikrobiell wirksamen Alkohol der Formel I 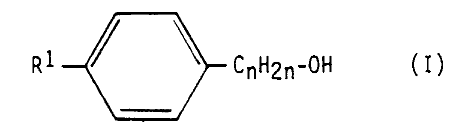 in der R¹ Wasserstoff oder eine Alkylgruppe mit 1 bis 4 C-Atomen und n eine ganze Zahl von 1 bis 6 ist und
(B) mindestens ein antimikrobiell wirksames 1,2- oder 1,3-Diol der Formel II
R²-CHOH-(CHR³)ₓ-CH₂OH (II)
in der x = 0 oder 1 ist und wenn x = 0 ist, R² eine Alkylgruppe mit 6 bis 22 C-Atomen oder eine Alkoxymethyl- oder 2-Hydroxy-alkoxymethylgruppe mit jeweils 6 bis 22 C-Atomen in der Alkoxygruppe ist und wenn x = 1 ist, die Gruppe R² Wasserstoff ist und R³ eine der vorgenannten Bedeutungen von R² hat, wobei die Komponenten (A) und (B) im Gewichtsverhältnis (A):(B) = 9:1 bis 1:9 vorliegen.

2. Antimikrobiell wirksame Gemische nach Anspruch 1, dadurch gekennzeichnet, daß als Komponent B antimikrobiell wirksame 1,2-Diole der Formel II enthalten sind, in der x = 0 und R² eine Alkyl- oder Alkoxymethylgruppe mit 8 bis 14 C-Atomen in der Alkyl- oder Alkoxygruppe ist.

3. Antimikrobiell wirksame Gemische nach Anspruch 1 oder 2, dadurch gekennzeichnet, daß als Komponente A ein aromatischer Alkohol der Formel I enthalten ist in der n = 2 oder 3 ist.

4. Verwendung der antimikrobiell wirksamen Gemische gemäß Anspruch 1 bis 3 zur Konservierung wäßriger Zubereitungen mikrobiell abbaubarer Stoffe.

5. Wäßrige Zubereitungen zur Reinigung und Pflege des Körpers mit einem Gehalt an mikrobiell abbaubaren Ölen, Fetten, Proteinen, Kohlenhydraten oder Derivaten davon, dadurch gekennzeichnet, daß zur Konservierung ein antimikrobiell wirksames Gemisch gemäß Anspruch 1 bis 3 in einer Menge enthalten ist, die einem Gehalt von 0,2 bis 5 Gew.-% der Summe der Komponenten (A) + (B) entspricht.

## Claims

1. Antimicrobial mixtures containing
(A) at least one antimicrobial alcohol corresponding to formula I: 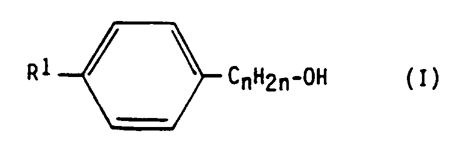 in which R¹ is hydrogen or a C₁₋₄ alkyl group and n is an integer of 1 to 6, and
(B) at least one antimicrobial 1,2- or 1,3-diol corresponding to formula II:
R²-CHOH-(CHR³)ₓ-CH₂OH (II)
in which x = 0 or 1 and, where x = 0, R² is a C₆₋₂₂ alkyl group or an alkoxymethyl or 2-hydroxyalkoxymethyl group containing 6 to 22 carbon atoms in the alkoxy group and, where x = 1, R² is hydrogen and R³ has one of the meanings mentioned above for R²,
components (A) and (B) being present in a ratio by weight of (A) to (B) of 9:1 to 1:9.

2. Antimicrobial mixtures as claimed in claim 1, characterized in that antimicrobial 1,2-diols corresponding to formula II, in which x = 0 and R² is an alkyl or alkoxymethyl group containing 8 to 14 carbon atoms in the alkyl or alkoxy group, are present as component B.

3. Antimicrobial mixtures as claimed in claim 1 or 2, characterized in that an aromatic alcohol corresponding to formula I, in which n = 2 or 3, is present as component A.

4. The use of the antimicrobially active mixtures claimed in claims 1 to 3 for the preservation of agueous preparations of microbially degradable substances.

5. Aqueous preparations for the cleansing and care of the body containing microbially degradable oils, fats, proteins, carbohydrates or derivatives thereof, characterized in that an antimicrobial mixture according to claims 1 to 3 is present for preservation in a quantity corresponding to a content of 0.2 to 5% by weight, based on the sum of components (A) + (B).

## Revendications

1. Mélanges antimicrobiens contenant
(A) au moins un alcool antimicrobien de formule I 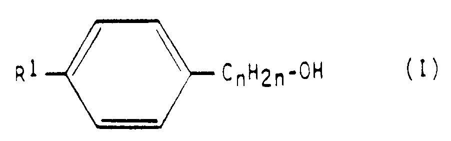 dans laquelle R¹ représente de l'hydrogène ou un groupe alkyle portant 1 à 4 atomes de C et n un nombre entier de 1 à 6 et
(B) au moins un 1,2- ou 1,3-diol antimicrobien de formule II
R²-CHOH-(CHR³)ₓ-CH₂OH (II)
dans laquelle x est égal à 0 ou 1 et si x = 0, R² représente un groupe alkyle portant 6 à 22 atomes de C ou un groupe alcoxyméthyle ou 2-hydroxyalcoxyméthyle portant chacun 6 à 22 atomes de C dans le groupe alcoxy et si x = 1, le groupe R² représente de l'hydrogène et R³ a l'une des significations précitées de R², les constituants (A) et (B) se trouvant dans un rapport pondéral (A):(B) allant de 9:1 à 1:9.

2. Mélanges antimicrobiens selon la revendication 1, caractérisés en ce que le constituant B consiste en 1,2-diols antimicrobiens de formule II dans laquelle x = 0 et R² est un groupe alkyle ou alcoxyméthyle comprenant 8 à 14 atomes de C dans le groupe alkyle ou alcoxy.

3. Mélanges antimicrobiens selon la revendication 1 ou 2, caractérisés en ce que le constituant A consiste en un alcool aromatique de formule I dans laquelle n = 2 ou 3.

4. Utilisation des mélanges antimicrobiens selon les revendications 1 à 3 pour conserver des préparations aqueuses à base de substances sujettes à une dégradation microbienne.

5. Préparations aqueuses de toilette et de soins corporels contenant des huiles, graisses, protéines ou glucides sujets à une dégradation microbienne ou leurs dérivés, caractérisées en ce qu'elles contiennent pour leur conservation un mélange antimicrobien selon les revendications 1 à 3 dans une quantité correspondant à une concentration de 0,2 à 5 % en poids de la somme des constituants (A) + (B).
